# EUROPÄISCHE PATENTANMELDUNG

(11) **EP 1 057 895 A1**
(43) Veröffentlichungstag der Anmeldung: **06.12.2000**
(21) Anmeldenummer: 99110759.0
(22) Anmeldetag: 04.06.1999
(51) Int. Cl.: C12N 15/62, C07K 14/245, A61K 38/16, C07K 16/12

(54) **Fusionsprotein das das Fragment B des Shigatoxins enthält, dieses enthaltende (Impf-)Stoffzusammensetzung und Verfahren zu dessen Herstellung**

(71) Anmelder: Lohmann Animal Health GmbH & Co. KG, 27472 Cuxhaven (DE)
(72) Erfinder: Baljer, Georg, Prof.Dr., 35452 Heuchelheim (DE); Franke, Silvia, Dr., 35396 Giessen (DE)
(74) Vertreter: Patentanwälte Hauck, Graalfs, Wehnert, Döring, Siemons

(57) **Zusammenfassung**

Rekombinantes Fusionsprotein mit einem subgenischen Stx2e-Fragment des Shiga Toxins 2e (Stx2e) in Fusion mit einem terminalen Tag, dessen Größe etwa der Größe des Fragmentes oder eines Bruchteils des Fragmentes entspricht.

## Beschreibung

Die Erfindung bezieht sich auf ein rekombinantes Fusionsprotein, auf eine (Impf-)Stoffzusammensetzung mit dem rekombinanten Fusionsprotein und auf ein Verfahren zur Herstellung des rekombinanten Fusionsproteins.

Die Ödemkrankheit der Schweine wird durch Shiga-Toxin bildende Escherichia Coli (STEC) hervorgerufen. Hauptvirulenzfaktor dieser Erreger und ausschließlich verantwortlich für die klinischen Symptome ist das Shiga Toxin 2e (Stx2e) (MacLeod et al. 1991) Da die Erkrankung in vielen Fällen einen perakuten Verlauf zeigt und Therapieversuche meist zu spät begonnen werden bzw. nicht den gewünschten Erfolg zeigen, wäre die Entwicklung einer wirksamen Prophylaxe wünschenswert. Die Gewinnung und Aufreinigung des Stx2e ist problematisch.

Die B-Untereinheit des Stx2e kommt aus verschiedenen Gründen als Vakzinekandidat in Frage. Sie wird von Seren rekonvaleszenter Ferkel erkannt, d.h. sie besitzt antigene Determinanten. Des weiteren induziert die B-Untereinheit des Toxins nach parenteraler Applikation die Bildung toxin-neutralisierender Antikörper (Acheson et al. 1996; Boyd et al. 1991). Mit Hilfe gentechnischer Methoden ist es gelungen, ein rekombinantes Fusionsprotein herzustellen, das aus einem Fragment der Stx2eB-Untereinheit und der Glutathion-S-Transferase von Shistosoma Japonicum besteht (Franke et al. 1995). Bei der Ödemkrankheit der Absetzferkel wurde bereits sowohl die Erregerausscheidung als auch die immunologische Reaktion auf die STEC-Infektion über einen längeren Zeitraum untersucht. Das zum Nachweis von Stx2e-Antikörpern eingesetzte rekombinante Fusionsprotein aus einem Fragment der Stx2eB-Untereinheit und der Glutathion-S-Transferase eignete sich sehr gut zum indirekten Nachweis der STEC-Infektion und wurde bislang als potentielles Impfantigen zur Prophylaxe der Ödemkrankheit angesehen (Wieler L. H., Franke Sylvia, Rose M. und Karch H. Charakterisierung der Immunantwort bei der Ödemkrankheit des Schweines mit einer rekombinanten B-Untereinheit des Shiga-like-Toxins-IIₑ. Vortrag gehalten auf dem 21. DVG Kongress in Bad Nauheim (März 1995)).

Davon ausgehend liegt der Erfindung die Aufgabe zugrunde, ein insbesondere für Impfzwecke geeignetes rekombinantes Fusionsprotein, ein dieses codierendes Plasmid, eine das Fusionsprotein enthaltende (Impf-)Stoffzusammensetzung für verschiedene Verwendungen im Zusammenhang mit der Ödemkrankheit, insbesondere der Schweine, und ein Verfahren zur Herstellung des rekombinanten Fusionsproteins zur Verfügung zu stellen.

Die Aufgabe wird durch ein rekombinantes Fusionsprotein mit den Merkmalen des Anspruchs 1, eine (Impf-)Stoffzusammensetzung mit den Merkmalen des Anspruchs 5, einen E.coli-Stamm gemäß Plasmid gemäß Anspruch 14 und durch ein Verfahren mit den Merkmalen des Anspruchs 16 gelöst. Ausgestaltungen der Erfindung sind in den Unteransprüchen angegeben.

Erfindungsgemäß werden ein rekombinantes Fusionsprotein und eine dieses enthaltende (Impf-)Stoffzusammensetzung zur Verfügung gestellt, die im Zusammenhang mit der Ödemkrankheit, insbesondere der Schweine, verschiedenen Verwendungen zugeführt werden können. So kommen insbesondere als Verwendungen in Betracht:
- der Nachweis von Antikörpern gegen Stx2e,
- die Diagnose der Ödemkrankheit,
- die Erzeugung monoklonaler Antikörper gegen das Toxin des Ödemkrankheitserregers, insbesondere als Basis für die Kontrolle der Ausbeute bei der Gewinnung des rekombinanten Fusionsproteins oder als Basis für die Gewinnung des Holotoxins durch immunaffinitätschromatografische Reinigung,
- die Immunisierung gegen die Ödemkrankheit, insbesondere der Schweine.

Das rekombinante Fusionsprotein ist ein subgenisches Stx2e-Fragment des Shiga-Toxins 2e in Fusion mit einem terminalen Tag, dessen Größe etwa der Größe des Fragments oder eines Bruchteils des Fragments entspricht. Der terminale Tag ist eine markierte Endgruppe in der Aminosäurenfolge des Proteins. Vorzugsweise ist das subgenische Stx2e-Fragment eine B-Untereinheit (Stx2eB) des Shiga Toxins 2e. Die Größe des terminalen Tags beträgt vorzugsweise maximal 5 kDa, weiterhin vorzugsweise maximal 1 kDa. Weiterhin vorzugsweise handelt es sich dabei um einen aminoterminalen His-Tag. Das His-Tag umfaßt sechs Histidine. Seine Größe beträgt ca. 0,66 kDa.

Das rekombinante Fusionsprotein weist wesentliche antigene Domänen des nativen Proteins auf, die seine Eignung für verschiedene Verwendungen im Zusammenhang mit der Ödemkrankheit begründen. Dies ist zwar theoretisch auch schon bei den bislang bekannten rekombinanten Fusionsproteinen aus einem Fragment der Stx2eB-Untereinheit und der Gluthathion-S-Transferase der Fall. Hier stellt sich allerdings das Problem, daß nach Einschätzung der Anmelderin mit störenden immunologischen Reaktionen gerechnet werden muß, die einem Einsatz der gattungsgemäßen Fusionsproteine zu z.B. therapeutischen Zwecken entgegenstehen. Ein wesentlicher Vorteil der erfindungsgemäßen Fusionsproteine besteht demgegenüber darin, daß hier aufgrund des speziell ausgewählten Tags nicht mit störenden Immunantworten zu rechnen ist und damit erstmals Fusionsproteine zur Verfügung stehen, die z.B. in Impfstoffen einsetzbar sind. Wie bei gattungsgemäßen Fusionsproteinen, so begünstigt auch das erfindungsgemäß verwendete Tag die Gewinnung des rekombinanten Fusionsproteins, insbesondere dessen Reinigung, beispielsweise durch ein affinitätschromatografisches Verfahren.

Von besonderer Bedeutung für die Impfstoffzusammensetzung ist die Auswahl des Adjuvans. Beispielsweise kann eine W/O/W- (z. B. ISA 206), eine W/O-Emulsion (z. B. iFA-inkomplettes Freund'sches Adjuvans, eine wäßrige Suspension (z. B. Aluminiumhydroxid) oder eine O/W-Emulsion zum Einsatz kommen.

Oligomere aus vernetzten His-Stx2eB-Monomeren können besonders wirksame Fusionsproteine bilden.

Nach dem erfindungsgemäßen Verfahren zur rekombinanten Herstellung eines subgenischen Fragments des Shiga Toxin 2e (Stx2e) in Fusion mit einem terminalen Tag wird in ein geeignetes Vektorsystem eine Untereinheit aus dem Stx2e-Operon kloniert, das entstandene rekombinante Plasmid in einen E. coli-Stamm transformiert, das entstandene Expressionssystem induziert und das Fusionsprotein exprimiert und gereinigt.

Das Gen der B-Untereinheit von Shiga Toxin 2e (Stx2eB) wurde in verschiedene Expressionsvektoren kloniert. Mit den so entstandenen rekombinanten Plasmiden wurden verschiedene E-coli-K12-Laborstämme transformiert. Alle Transformanden wurden in Expressionsstudien unter variierenden Bedingungen (Temperatur, Induktionslevel, Induktionsdauer) auf Bildung der rekombinanten B-Untereinheit getestet. Der Transformant bzw. Klon mit der höchsten Ausbeute an rekombinantem Protein im Verhältnis zum Gesamtzellproteingehalt wurde ermittelt. Für das von diesem Stamm gebildete Fusionsprotein, bestehend aus der reifen B-Untereinheit mit einem N-terminalen His-Tag (His-Stx2eB), wurde ein Reinigungsverfahren entwickelt und im Labormaßstab getestet. Für die Umsetzung des Reinigungsverfahrens im Großmaßstab ist FPLC unter Verwendung geeigneter Puffersysteme vorgesehen.

### Beispiel

### Herstellung der rekombinanten B-Untereinheit des Stx2e

Für die Herstellung der rekombinanten B-Untereinheit wird der Stamm E. coli Cux-Stx2eB, DSM-Nr. 12721 (Deutsche Sammlung von Mikroorganismen und Zellkulturen GmbH, Mascheroder Weg 1 b, D-38124 Braunschweig), verwendet.
Dieser E. coli-Laborstamm enthält das Plasmid pHIT-24, welches die B-Untereinheit des Stx2e kloniert.
Von diesem Stamm wurde ein seed lot System angelegt und bei -78°C , abgefüllt in 2 ml Kryo-Vials, gelagert.
Für die Produktion der rekombinanten B-Untereinheit wird 1 Ampulle working seed (2 ml) für die Anzucht einer Vorkultur 1 aufgetaut.
Die Vorkultur 1 wird unter folgenden Bedingungen hergestellt:
- Medium :: 150 ml sterile Standard-I-Nährbouillon + 0,01% Ampicillin in einem Erlenmeyerkolben 300 ml
- Bebrütung :: 15 Stunden bei 37 °C, Standkultur

Für den Hauptansatz wird ein Fermenter "BIOSTAT B" mit einem 5-L-Kulturgefäß benutzt. Dieses Gefäß ist mit 4 L Standard-I-Nährbouillon + 0,01% Ampicillin gefüllt und wurde als Einheit für 25 Minuten bei 121 °C autoklaviert..
In dieses Medium wird die Vorkultur 1 gegeben und unter folgenden Bedingungen für 6 Stunden kultiviert :
Temperatur : 37 °C
pH = 7,0-7,1
Rührgeschwindigkeit: 100-150 rpm
Luftzufuhr : 2 L / min
Die Regulierung des pH wird durch das automatische Zugeben einer sterilen 10 % NaOH-Lösung gewährleistet.

Die Induktion wurde nach 6 Stunden Kultivierung durch Zugabe von 0,25 mM IPTG-Lösung * und einem pH-Sprung von 7,1 auf 7,5 eingeleitet.Die Induktionsdauer betrug ca. 3,5 Stunden.
* IPTG: Isopropylbeta-D-Thiogalaktopyranosit

Anschließend wurde die Kultur in ein 10-L-Erntebehälter gepumpt und in einer Zentrifuge mit 2500 x g abzentrifugiert. Der Überstand wurde verworfen, das Pellet in 200 ml eines 8 M
Harnstoffpuffers aufgenommen und für ca. 15 Stunden in einem Kühlraum (4-8 °C) verbracht.
Das resuspendierte Pellet wurde danach mit Ultraschall behandelt (4 x 15 Minuten, 190 Hertz, 0,3 Sekunden pulsierend) und anschließend mit 10 000 x g zentrifugiert. Der Überstand wurde vorsichtig entnommen und diente der weiteren Bearbeitung; das bei diesem Schritt erhaltene Pellet wurde verworfen.
Im Anschluß daran wurde die Lösung mittels einer Ultrafiltration ("Pellicon XL ") von 200 ml auf 80 ml eingeengt.

Die so erhaltene 80 ml-Proteinlösung wurde dann mittels einer Affinitäts-Chromatographie (FPLC "Äktaexplorer") weiter bearbeitet.
Das das rekombinante Zielprotein enthaltene Material wurde fraktioniert, jeweils 3 ml, aufgetragen und über eine mit Metall-Chelat-Matrix (Ni-NTA, Qiagen) beladene Säule (Volumen : 8 ml) gegeben.
Diese Matrix bindet spezifisch das His-Tag des rekombinanten Proteins.
Das Targetprotein wird vom Metall festgehalten und unter denaturierenden Bedingungen (8 M Harnstoff, 0,1 M Na₂HPO₄, 10 mM Tris/HCL, pH 8) gewaschen.
Nach der Entfernung der kontaminierenden Proteine wird das rekombinante Protein durch einen pH-Sprung (8 M Harnstoff, 0,1 M NaH₂PO₄, 10 mM Tris/HCL, pH 3) von der Affinitätsmatrix desorbiert und am Säulenausgang aufgefangen.

Das gereinigte Protein wird mittels Cross-Flow-Filtration (Porengröße 5 kDa) konzentriert.
Nach Prüfung der Reinheit und Ausbeute (über SDS-Gelelektrophorese, Westernblotting, Elisa, Proteinbestimmung) erfolgt der Austausch des Harnstoff-Puffers gegen eine physiologische Pufferlösung (PBS, pH 7,2). Der Austausch erfolgt mittels Cross-Flow-Filtration (Porengröße 5 kDa).
Das rekombinante Protein lag in einer Konzentration von 300 µg/ml vor.

### Beschreibung des rekombinanten Fusionproteins

Das Targetprotein wird durch das Genfragment Stx2eB codiert. Die Größe dieses subgenischen Fragments der B-Untereinheit von Stx2e beträgt 228 bp.
Folgende eigenschaften des rekombinanten Proteins wurden geprüft :
1. Molekulargewichtsgröße
   Das Targetprotein hat ein in der SDS-Gelelektrophorese ermittels Molekulargewicht von ca. 7,5 kDa.
2. Kontrolle des rekombinanten Proteins im Immunoblot mit Seren erkrankter Ferkel
   Das gereinigte Antigen wurde im Immunoblot mit Seren von an Ödemkrankheit erkrankten Ferkeln untersucht.Bei den Tieren handelte es sich um Ferkel aus Schweinezuchtbetrieben, in denen klinisch manifeste Erkrankungen mit Stx2e-E.coli-Stämmen auftraten.
   Über 90 % dieser Seren reagierten positiv mit dem rekombinanten Protein. Um falsch positive Ergebnise auszuschließen, wurden die Untersuchungen mit der B-Untereinheit, an die Glutathion-S-Transferase von Schistosoma japonicum gekoppelt, verifiziert.
3. Kontrolle des rekombinanten Proteins mit monoklonalen Antikörpern gegen Stx2eB
   Um festzustellen, ob die Konformation der rekombinanten B-Untereinheit dem Wildtyp-Protein ähnelt, wurde das rekombinante Stx2eB im Dot-Blot-Verfahren untersucht.
   Dazu wurde der monoklonale Antikörper BC5BB12 eingesetzt, der spezifisch die B-Untereinheit von Stx2 erkennt und auch mit der B-Untereinheit von Stx2e kreuzreagiert.
   Als Positivkontrolle wurde das Stx2e-Holotoxin mitgeführt. Als Negativkontrolle diente eine Rohtoxinpreparation von Stx1.
   Der monoklonale Antikörper BC5BB12 reagierte sowohl mit dem Stx2e-Holotoxin, als auch mit dem rekombinanten Stx2eB-Protein, jedoch nicht mit dem Stxl.
4. Prüfung des rekombinanten Proteins auf Zytotoxizität im Verozelltest
   Die Zytotoxität des rekombinanten Proteins wurde im Zytotoxizitätstest an Verozellen, Helazellen sowie MDBK-Zellen untersucht. Zu diesem Zweck wurden Konzentrationen von 0,3 µg/ml bis 100 µg/ml an rekombinanten Stx2eB eingesetzt. Selbst in den niedrigsten Verdünnungsstufen konnte in keiner der untersuchten Zelllinien ein signifikanter Unterschied zur Negativkontrolle (Puffer ohne rekombinantes Protein) festgestellt werden. Diese Ergebnisse bestätigen, daß das rekombinante Stx2eB per se nicht zytotoxisch ist.
5. Nachweis der Immunogenität des rekombinanten Stx2eB im Kaninchenversuch.
   Zwei weibliche Kaninchen derRasse "Weiße Neuseeländer" im Alter von ca. 12 Monanten wurden mit dem rekombinanten Stx2eB immunisiert. Bei der 1. Vakzination wurden 100 µg Antigen unter Zugabe von inkompletten Freundschen Adjuvans (iFA) subcutan verabreicht.Die Boosterung erfolgte sechs Wochen später mit 50 µg rekombinantem Stx2eB subcutan, ebenfalls mit iFA. Die vor und nach der Vakzinierung gewonnen Seren wurden im Immunoblot untersucht.
   In beiden Kaninchen wurde eine spezifische Serumkonversion nachgewiesen.

### Beschreibung der Herstellung von Vakzineformulierungen (Beispiele)

1. Herstellung einer W/O/W-Vakzineformulierung
   Unter sterilen Bedingungen wird das Antigen als wäßrige Phase (Temperatur 22°C) während des Rührens (U.T.< 2000 Upm) kontinuierlich in das Adjuvans (z. B. Montanide ISA 206) gegeben. Anschließend wird die Emulsion 10 minuten bei ca. 2000 Upm homogenisiert.
   Nach 24 h Lagerungszeit bei 8°C wird die Vakzineformulierung erneut homogenisiert. Die Phasenlage wird mikroskopisch und im Färbetest geprüft.
2. Herstellung einer W/O-Vakzineformulierung
   Unter sterilen Bedingungen wird das Antigen als wäßrige Phase (Temperatur 22°C) während des Rührens (U.T. < 2000 Upm) kontinuierlich in das Adjuvans (z. B. inkomplettes Freundsches Adjuvans) gegeben und emulgiert.
   Die Phasenlage wird mikroskopisch und im Färbetest geprüft.
3. Herstellung einer wäßrigen Suspension
   Unter sterilen Bedingungen wird das wäßrige Antigen während des Rührens (z.B. Magnetrührer) kontinuierlich in das wäßrige Adjuvans (z. B. Aluminiumhydroxide) gegeben und gerührt.
   Die Vakzine wird geprüft anhand der Parameter pH und Tonizität.
4. Herstellung einer O/W-Suspension
   Unter sterilen Bedingungen wird das wäßrige Antigen kontinuierlich in das Adjuvans gegeben und emulgiert.
   Die Phasenlage wird mikroskopisch und im Färbetest geprüft.

Nach der Herstellung der Vakzineformulierungen wurde diese bis zu ihrer weiteren Verwendung im Kühlschrank bei Temperaturen von +4°C - +8°C gelagert.

### Beispiel des Nachweises der immunogenen Wirkung des rekombinanten Stx2eB im Zieltier Schwein unter Verwendung verschiedener Vakzineformulierungen

### Ziel des Versuches

Untersuchung des Fragestellung : Kann das gentechnisch hergestellte rekombinante Stx2eB-Protein (unter Verwendung verschiedener Adjuvantien) nach zweimaliger i.m. Applikation im Absatzferkel eine Immunantwort induzieren ?

Für diesen Versuch wurden 8 Absetzer im Alter von 6 Wochen eingesetzt.
6 Tiere wurden mit Vakzinepräparationen behandelt, 2 Tiere erhielten ein Placebo verabreicht. Die Impfstoffapplikationen erfolgten zweimal im Abstand von 3 Wochen.
Von jedem Tier wurden folgende Blutproben entnommen .
1. Vor der 1. Immunisierung
2. 14 Tage nach der 1. Immunisierung
3. Unmittelbar vor der 2. Immunisierung (21 Tage nach der 1. Immunisierung)
4. 14 Tage nach der 2. Immunisierung
5. 21 Tage nach der 2. Immunisierung

Die Seren wurden im Elisa auf das Vorhandensein von spezifischen Antikörpern untersucht, die gegen das rekombinante Stx2eB gerichtet sind.

Außerdem wurden die Verträglichkeit und Sicherheit der Vakzine bewertet.

### Allgemeine Versuchsdaten

### Tiere

Tierart : Schwein
Tierkategorie : Absetzer
Alter : 6 Wochen (bei 1. Impfung)
Geschlecht : gemischt
Immunstatus der Tiere bei Versuchsbeginn : Stx2eB-Antikörper negativ
Haltungsform : Gruppenhaltung
Fütterungsschema : ad libitum
Wasserversorgung : aus Wasserleitung ad libitum
Einsatz von Futterzusatzstoffen : kein Einsatz von Futterzusatzstoffen

### Impfstoffverabreichungsparameter

Applikationsweise : Injektion
Applikationsweg : i. m.
Zeitraum zwischen den zwei Impfstoffapplikationen : 3 Wochen
Vorbehandlung des verabreichten Mittels : keine
Vorbehandlung der Versuchstiere : keine
Anzahl der Impflinge : 8
Anzahl der Kontrolltiere : 2

Studiendesign : randomisiert, blind

### Impfstoffdosis, Tierkennzeichnung, Vakzineeinsatz

Der konkrete Versuchsplan ist in Tabelle 1 dargestellt.

### Adjuvansschlüssel

Adjuvans A - ISA 206
Adjuvans B - iFA
Adjuvans C - Montanide

### Versuchsverlauf

Auftreten von Nebenwirkungen :
- nach 1. Impfung -: leichte Beeinflußung des Allgemeinzustandes und der Futteraufnahme,
Tier 7 und 8 hatten eine leichte Körpertemperaturerhöhung + leichten Durchfall
- nach 2. Impfung -: außer Körpertemperaturerhöhung beim Tier 8 keine weiteren Nebenwirkungen

Anzahl der Tiere, die während des Versuches ausschieden :
Asetzer Nr. 2, Grund : E.coli-verursachte Magen - und Darmentzündung

Auftreten von Krankheiten, die nicht mit der Vakzination zusammenhängen :
außer der Erkrankung beim Absetzer Nr. 2 keine

Behandlung mit anderen Mitteln : keine

### Ergebnisse

Verträglichkeit und Sicherheit der Vakzineformulierungen :
Die Vakzineformulierungen können insgesamt als verträglich und sicher eingeschätzt werden, obwohl es nach der 1. Immunisierung zu einer leichten Störung des Allgemeinbefindens, zu einer kurzzeitigen Beeinträchtigung der Futteraufnahme und zu einer Körpertemperturerhöhung - verbunden mit leichten Durchfall - bei den Tieren 7 und 8 kam.Die 2. Impfung wurde ohne das Auftreten von klinischen Symptomen vertragen. Nur das Tier Nr. 8 reagierte auf die erneute Imstoffapplikation mit einer Erhöhung der Körpertemperatur.
Lokale Gewebsreaktionen - palpatorisch bemerkbares leichtes Ödem - traten nur nach der 1. Impfung an der Injektionsstelle der Tiere 5 und 6 auf.
Nach der Schlachtung der Ferkel konnten an den Injektionsstellen, mit Ausnahme der Tiere 1 und 3, makroskopisch erkennbare Entzündungen um den Strichkanal, die mit nekrotischem Material bei allen Tieren ausgefüllt war, festgestellt werden. Bei der histologischen Untersuchung der Ferkel 1 und 3 konnte nur eine leichte Bindegewebsproliferation (Angioblasten mit Infiltration von Lymphozyten und Histiozyten) festgestellt werden, während der mit nekrotischem Material ausgefüllte Stichkanal bei allen anderen Ferkeln mit einer bindegewebigen Kapsel umgegeben war. In der bindegewebigen Kapsel konnte eine Entzündung mit Infiltration von Lymphozyten und Histiozyten beobachtet werden.

### Wirksamkeit der Vakzineformulierungen

Mit diesem Versuch konnte gezeigt werden, daß das rekombinante Stx2eB nach i. m.
Applikation in 6 Wochen alten Absetzern vom Immunsystem der Tiere erkannt wird und eine Immunantwort - Produktion von spezifischen Immunglobulinen - induziert.
Der Nachweis dieser Antikörper wurde mittels Elisa und Immunoblot durchgeführt. Die Stärke der Immunantwort scheint von der Wahl der verwendeten Vakzineformulierung abhängig zu sein.
Unter den gewählten Versuchsbedingungen konnten die besten Ergebnisse mit einer W/O-Emulsion (z. B. unter Verwendung von iFA) erziehlt werden.Im einzelnen sind die Ergebnisse in Tabelle 2 dargestellt.

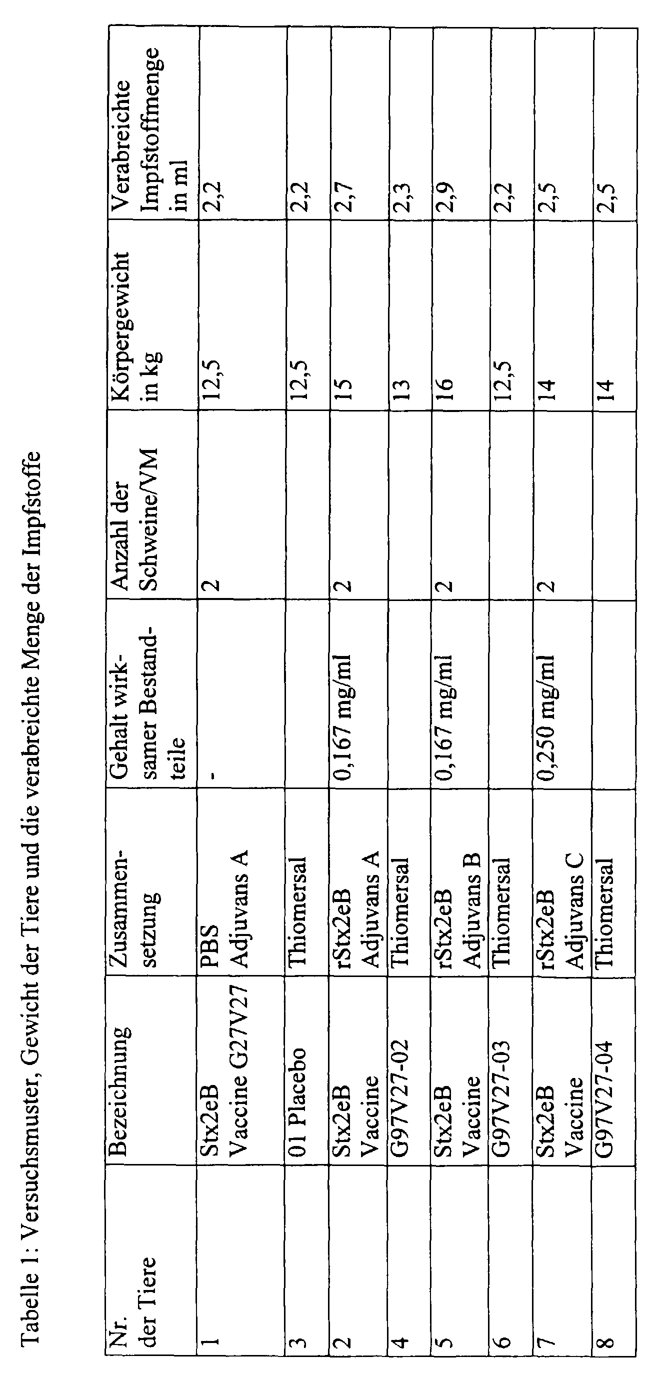

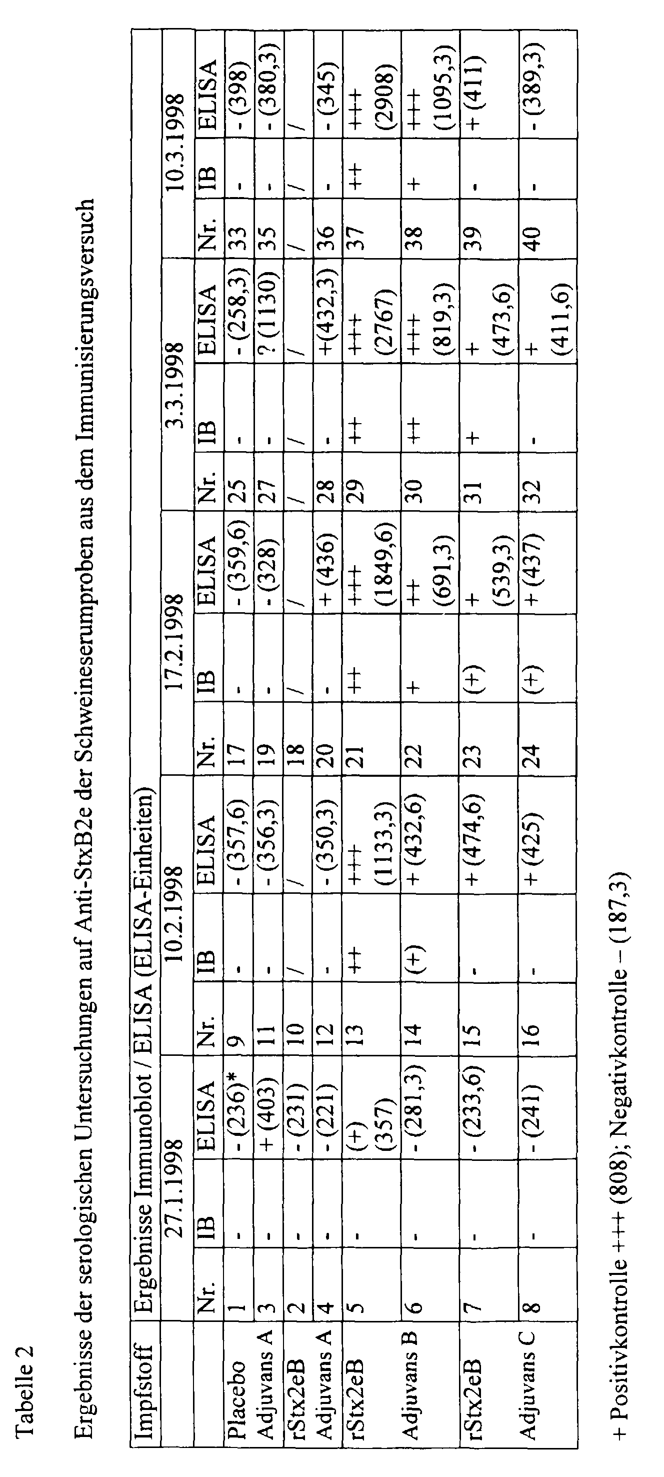

Hinterlegter Mikroorganismus:

Der E.Coli-Stamm Cux-Stx2eB wurde hinterlegt unter der unsprünglichen Bereichung Cux-SLT-IIₑ-B bei der
DSMZ-Deutsche Sammlung von Mikroorganismen und Zellkulturen GmbH, Mascheroder Weg 1b, D-38124 Braunschweig.

Ihm wurde von der Hinterlegungsstelle die Eingangsnummer
DSM 12721 zugeteilt.

## Patentansprüche

1. Rekombinantes Fusionsprotein mit einem subgenischen Stx2e-Fragment des Shiga Toxins 2e (Stx2e) in Fusion mit einem terminalen Tag, dessen Größe etwa der Größe des Fragmentes oder eines Bruchteils des Fragmentes entspricht.

2. Rekombinantes Fusionsprotein nach Anspruch 1, bei der das subgenische Stx2e-Fragment eine B-Untereinheit (Stx2eB) des Shiga Toxins 2e ist.

3. Rekombinantes Fusionsprotein nach Anspruch 1 oder 2 mit einer Größe des terminalen Tags von maximal 1 kDa.

4. Rekombinantes Fusionsprotein nach einem der Ansprüche 1 bis 3 mit einem aminoterminalen His-Tag.

5. Rekombinantes Fusionsprotein nach einem der Ansprüche 1 bis 4 mit mehreren vernetzten Fusionsproteinen.

6. (Impf-)Stoffzusammensetzung für verschiedene Verwendungen im Zusammenhang mit der Ödemkrankheit der Tiere, insbesondere Säugetiere, vor allem Schweine, mit einem subgenischen Fragment des Shiga Toxins 2e (Stx2e) in Fusion mit einem terminalen Tag, dessen Größe etwa der Größe des Fragmentes oder eines Bruchteils des Fragmentes entspricht.

7. (Impf-)Stoffzusammensetzung nach Anspruch 6, bei der das subgenische Stx2e-Fragment eine B-Untereinheit (STx2eB) des Shiga Toxins 2e ist.

8. (Impf-)Stoffzusammensetzung nach Anspruch 6 oder 7 mit einer Größe des terminalen Tags von maximal 1kDa.

9. (Impf-)Stoffzusammensetzung nach einem der Ansprüche 6 bis 8 mit einem aminoterminalen His-Tag.

10. (Impf-)Stoffzusammensetzung nach einem der Ansprüche 6 bis 9 mit mehreren vernetzten Fusionsproteinen.

11. (Impf-)Stoffzusammensetzung mit einem subgenischen Stx2e-Fragment des Shiga Toxins 2e, insbesondere nach einem der Ansprüche 6 bis 10, in einer W/O/W-Emulsion, in einer O/W-Emulsion, in einer W/O-Emulsion oder in einer wäßrigen Suspension.

12. (Impf-)Stoffzusammensetzung nach Anspruch 11 mit inkomplettem Freund'schen Adjuvans (iFA).

13. Plasmid enthaltend DNA, die ein Fusionsprotein nach einem der Ansprüche 1 bis 6 codiert.

14. E.coli-STamm, transformiert mit einem Plasmid gemäß Anspruch 13.

15. E.coli-Stamm gemäß Anspruch 14, hinterlegt bei der DSMZ - Deutsche Sammung von Mikroorganismen und Zellkulturen GmbH unter der Nummer DSM 12721.

16. Verfahren zur rekombinanten Herstellung eines subgenischen Fragmentes des Shiga Toxins 2e (Stx2e) in Fusion mit einem terminalen Tag, bei dem in ein geeignetes Vektorsystem eine Untereinheit aus dem Stx2e-Operon kloniert wird, das entstandene rekombinante Plasmid in einen E.coli-Stamm transformiert wird, das entstandene Expressionssystem induziert wird und das Fusionsprotein exprimiert und gereinigt wird.

17. Verfahren nach Anspruch 16, bei dem das subgenische Fragment eine B-Untereinheit (Stx2eB) des Shiga Toxins 2e ist.

18. Verfahren nach Anspruch 16 oder 17, bei dem das terminale Tag eine Größe von maximal 1kDa hat.

19. Verfahren nach einem der Ansprüche 16 bis 18, bei dem das terminale Tag ein aminoterminales His-Tag ist.

20. Verfahren nach einem der Ansprüche 16 bis 19, bei dem die Expressionskultur einer Lysepufferbehandlung unterzogen wird.

21. Verfahren nach einem der Ansprüche 16 bis 20, bei dem die Expressionskultur einer Behandlung in einer French Press oder mittels Ultraschall unterzogen wird.

22. Verfahren nach einem der Ansprüche 16 bis 21, bei dem die Expressionskultur nach Behandlung mit der French Press oder mittels Ultraschall und/oder mit Lysepuffer einer affinitätschromatografischen Reinigung zugeführt wird.

23. Verfahren nach Anspruch 22, bei dem die Reinigung mittels einer FPLC durchgeführt wird.

24. Verfahren nach einem der Ansprüche 16 bis 23, bei dem das gereinigte Fusionsprotein vernetzt wird.

25. Verfahren nach einem der Ansprüche 16 bis 24, bei dem durch Fusion von Milzzellen von unter Verwendung des rekombinanten Fusionsproteins immunisierten Mäusen mit Myelomzellen Hybridoma-Klone für die Herstellung von Anti-Stx2eB-Immunglobulinen erzeugt werden.

26. Verfahren nach Anspruch 25, bei dem die mittels der Hybridoma-Klone produzierten Antikörper zur in-process-Kontrolle für die Produktion der rekombinanten Fusionsproteine verwendet werden.

27. Verfahren nach Anspruch 25 oder 26, bei dem die von den Hybridoma-Klonen produzierten Antikörper für ein immunaffinitätschromatografisches Reinigungsverfahren für das Stx2e-Holotoxin verwendet werden.
